# EUROPEAN PATENT APPLICATION

(11) **EP 0 917 865 A1**
(43) Date of publication of application: **26.05.1999**
(21) Application number: 97309182.0
(22) Date of filing: 14.11.1997
(51) Int. Cl.: A61F 9/02

(54) **Protective eyewear with transparent lens unit having darkening capability**

(71) Applicant: Wang-Lee, Min-Young, Yung-Kang City, Tainan Hsien (TW)
(72) Inventor: Wang-Lee, Min-Young, Yung-Kang City, Tainan Hsien (TW)
(74) Representative: Alexander, Thomas Bruce

(57) **Abstract**

A protective eyewear includes a lens frame (2, 3a) and a transparent lens unit mounted on the lens frame (2, 3a). The lens unit includes at least one liquid crystal cell (40). A control circuit (5) is mounted on the lens frame (2, 3a) and is connected electrically to the liquid crystal cell (40). The control circuit (5) is operable so as to apply a control signal to the liquid crystal cell (40) to darken the lens unit in order to provide protection against strong ambient light.

## Description

The invention relates to protective eyewear, more particularly to a protective eyewear which includes a transparent lens unit that is capable of darkening in accordance with the strength of ambient light.

Protective eyewear, such as sunglasses and helmets for welding, usually include a dark-colored filtered lens unit which is designed to protect the user's eyes from strong light or infra-red light. However, since the darkness of the lens unit employed in the conventional protective eyewear is fixed, the conventional protective eyewear can only be used in strong ambient light conditions because the dark-colored filtered lens unit can cause poor visibility to the user in the presence of relatively poor ambient light.

Therefore, the object of the present invention is to provide a protective eyewear which includes a transparent lens unit that is capable of darkening in accordance with the strength of ambient light, thereby permitting use of the protective eyewear regardless of the ambient light conditions.

Accordingly, the protective eyewear of the present invention comprises: a lens frame; a transparent lens unit mounted on the lens frame, the lens unit including at least one liquid crystal cell; and a control circuit mounted on the lens frame and connected electrically to the liquid crystal cell, the control circuit being operable so as to apply a control signal to the liquid crystal cell to darken the lens unit in order to provide protection against strong ambient light.

Preferably, the control circuit comprises: a light sensing circuit for generating a detected light signal that varies according to strength of ambient light; a liquid crystal cell driver connected electrically to the light sensing circuit and the liquid crystal cell, the liquid crystal cell driver automatically generating the control signal that is applied to the liquid crystal cell according to the detected light signal from the light sensing circuit; and comparing means which interconnects electrically the light sensing circuit and the liquid crystal cell driver, the comparing means receiving the detected light signal from the light sensing circuit and generating an actuating signal for activating the liquid crystal cell driver to generate the control signal in order to vary darkness of the liquid crystal cell based on results of a comparison between the detected light signal and a pre-set reference signal.

In a preferred embodiment, the lens unit includes a pair of the liquid crystal cells. The lens frame includes a frame body which has a bottom portion that is formed with a nose bridge, and opposite side portions that have a temple mounted pivotally and respectively thereon. The frame body confines a lens opening unit which includes a pair of window openings. The liquid crystal cells are mounted on the frame body in a respective one of the window openings. The control circuit is connected electrically to the liquid crystal cells. The lens unit further includes a pair of optical filters, each of which is mounted on a respective one of the liquid crystal cells to provide protection against non-visible light.

In another preferred embodiment, the lens frame includes a helmet body that is formed with a window opening. The lens unit is mounted on the helmet body in the window opening.

Other features and advantages of the present invention will become apparent in the following detailed description of the preferred embodiments with reference to the accompanying drawings, of which:
Figure 1 is a perspective view of the first preferred embodiment of a protective eyewear in accordance with the present invention;
Figure 2 is a sectional view of the first preferred embodiment, taken along line II-II in Figure 1;
Figure 3 is a plot which illustrates how the darkness of a liquid crystal cell varies in response to an applied voltage;
Figure 4 is a schematic block diagram of a control circuit of the first preferred embodiment;
Figure 5 is a sectional view of the second preferred embodiment of a protective eyewear in accordance with the present invention; and
Figure 6 is a perspective view of the third preferred embodiment of a protective eyewear in accordance with the present invention.

Before the present invention is described in greater detail, it should be noted that like elements are denoted by the same reference numerals throughout the disclosure.

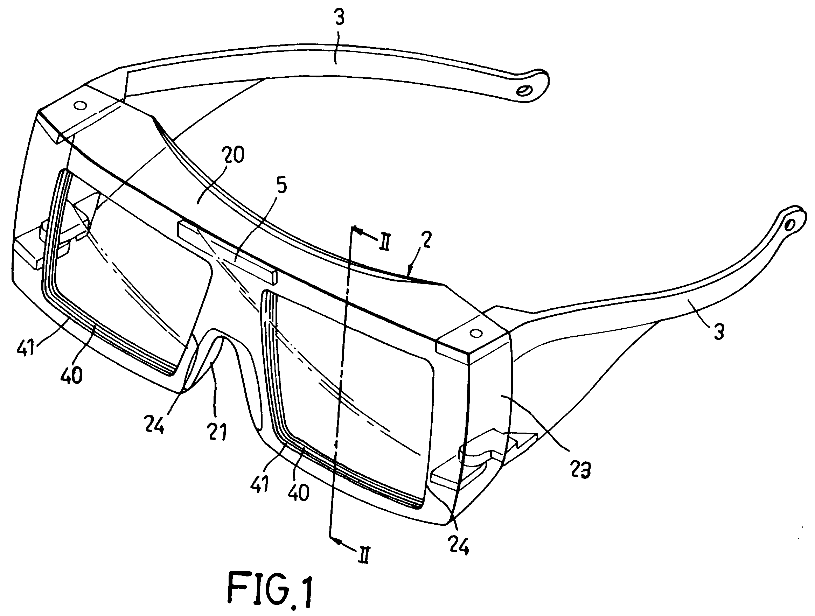

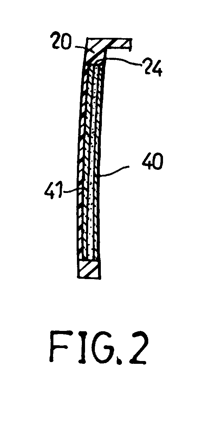

Referring to Figures 1 and 2, the first preferred embodiment of a protective eyewear according to the present invention is shown to be in the form of a pair of eyeglasses and comprises a lens frame 2, a transparent lens unit including a pair of liquid crystal cells 40 and a pair of optical filters 41, and a control circuit 5.

The lens frame 2 includes a frame body 20 which has a bottom portion that is formed with a nose bridge 21, and opposite side portions that are formed with a pair of side flanges 23. A pair of temples 3 are mounted pivotally and respectively on the side flanges 23. The frame body 20 confines a lens opening unit for mounting of the transparent lens unit therein. In this embodiment, the lens opening unit includes a pair of window openings 24.

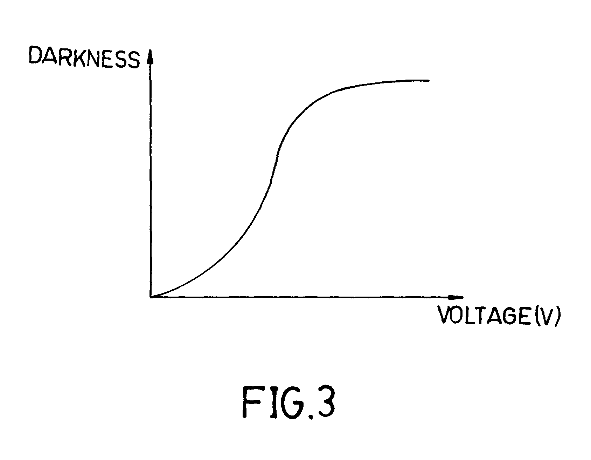

Each of the liquid crystal cells 40 is mounted on the frame body 20 in a respective one of the window openings 24. As is known in the art, the darkness of the liquid crystal cell 40 varies in accordance with the presence of an applied electric field. Figure 3 is a curve which illustrates how the liquid crystal cell 40 darkens in response to an applied voltage. As shown, the shade of the liquid crystal cell 40 darkens as the applied voltage increases. However, the darkness of the liquid crystal cell 40 becomes constant once the applied voltage reaches a certain threshold voltage.

Each of the optical filters 41 is mounted on one side of a respective one of the liquid crystal cells 40 and serves to filter out harmful non-visible light, such as infra-red light. Thus, only visible light can pass through the liquid crystal cells 40.

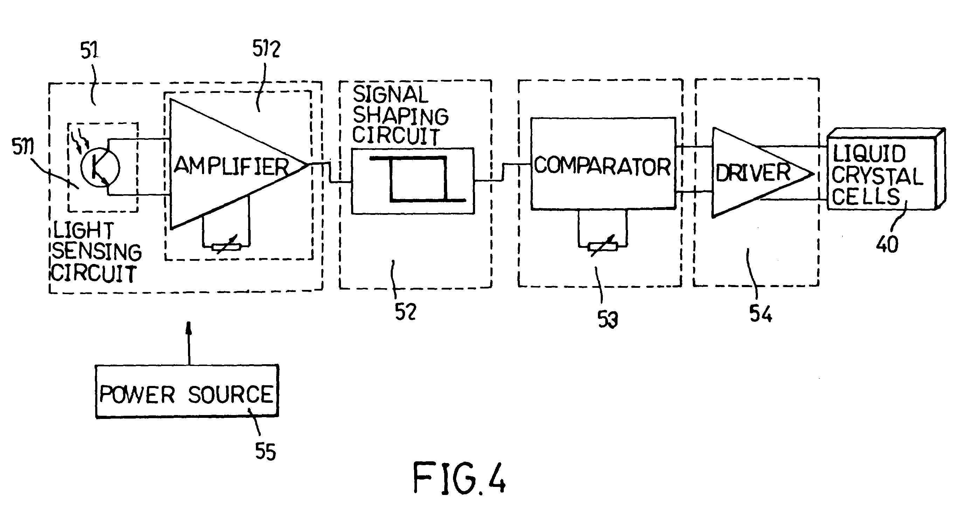

Referring once more to Figure 1, the control circuit 5 is mounted on an inner side of the top portion of the frame body 20. The control circuit 5 is connected electrically to the liquid crystal cells 40 and is operable so as to apply a control signal thereto in response to the strength of ambient light so as to enable the liquid crystal cells 40 to darken accordingly. As shown in Figure 4, the control circuit 5 includes a light sensing circuit 51, a comparing unit constituted by a signal shaping circuit 52 and a comparator 53, a liquid crystal cell driver 54 and a power source 55.

The light sensing circuit 51 includes a photo sensor 511, such as a phototransistor, and a signal amplifier 512 connected electrically to the photo sensor 511. The photo sensor 511 generates an electrical signal that varies according to the strength of ambient light and that is amplified by the signal amplifier 512 to obtain a detected light signal.

The signal shaping circuit 52 is connected electrically to the signal amplifier 512 and receives the detected light signal therefrom. The signal shaping circuit 52 processes the detected light signal to obtain a light data signal.

The comparator 53 is connected electrically to the signal shaping circuit 52 and receives the light data signal therefrom. The comparator 53 compares the light data signal with a pre-set reference signal, and generates an actuating signal based on the results of the comparison.

The liquid crystal cell driver 54 is connected electrically to the comparator 53 and the liquid crystal cells 40. The liquid crystal cell driver 54 is activated by the actuating signal from the comparator 53 so as to generate automatically the control signal that is applied to the liquid crystal cells 40 for varying the darkness of the latter according to the strength of ambient light as detected by the light sensing circuit 51.

The power source 55, such as a lithium cell, provides the electrical power that is required to operate the control circuit 5.

In operation, when the light sensing circuit 51 detects the presence of strong ambient light, the comparator 53 generates the actuating signal, thereby activating the liquid crystal cell driver 54 to generate, in turn, the control signal that is received by the liquid crystal cells 40, thus causing the latter to darken. At this time, the liquid crystal cells 40 cooperate with the optical filters 41 to protect the user's eyes from both strong light and non-visible light. Accordingly, when the light sensing circuit 51 detects the presence of weak ambient light, the comparator 53 generates another actuating signal which causes the liquid crystal cell driver 54 to lighten the liquid crystal cells 40, thereby preventing the latter from hindering visibility of the user in the presence of poor ambient light.

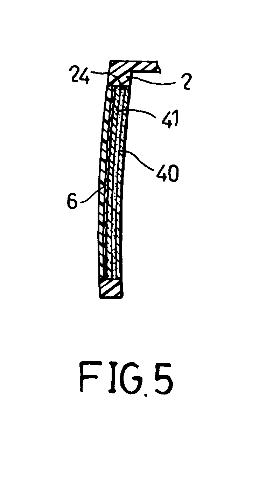

Figure 5 illustrates the second preferred embodiment of a protective eyewear according to the present invention. The second preferred embodiment is generally similar to the previous embodiment, except for the addition of a transparent protective piece 6 mounted on one side of each of the optical filters 41 opposite to the respective one of the liquid crystal cells 40 in order to prolong the service life of the optical filters 41.

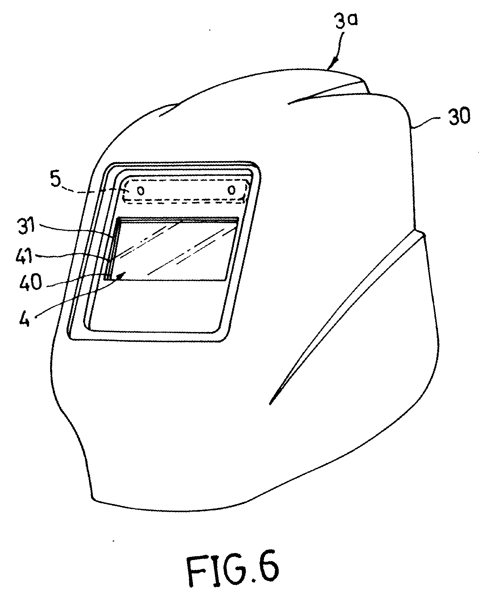

Figure 6 illustrates the third preferred embodiment of a protective eyewear according to the present invention. Unlike the previous embodiments, the third preferred embodiment is shown to be in the form of a welding helmet and includes a lens frame 3a, a transparent lens unit 4 including a liquid crystal cell 40 and an optical filter 41, and a control circuit 5.

The lens frame 3a includes a helmet body 30 that is formed with a window opening 31. The liquid crystal cell 40 is mounted on the helmet body 30 in the window opening 31. The optical filter 41 is mounted on one side of the liquid crystal cell 40. The control circuit 5 is mounted on an inner side of the helmet body 30 and is connected electrically to the liquid crystal cell 40.

The operation of the third preferred embodiment is generally similar to that of the previous embodiments and will not be detailed further.

## Claims

1. A protective eyewear including a lens frame (2, 3a) and a transparent lens unit mounted on said lens frame (2, 3a), characterized by said lens unit including at least one liquid crystal cell (40), and a control circuit (5) mounted on said lens frame (2, 3a) and connected electrically to said liquid crystal cell (40), said control circuit (5) being operable so as to apply a control signal to said liquid crystal cell (40) to darken said lens unit in order to provide protection against strong ambient light.

2. The protective eyewear of Claim 1, characterized in that said lens unit further includes an optical filter (41) mounted on said liquid crystal cell (40) to provide protection against non-visible light.

3. The protective eyewear of Claim 2, characterized in that said lens unit further includes a transparent protective piece (6) mounted on one side of said optical filter (41) opposite to said liquid crystal cell (40).

4. The protective eyewear of Claim 1, characterized in that said lens unit includes a pair of said liquid crystal cells (40), said lens frame (2) including a frame body (20) which has a bottom portion that is formed with a nose bridge (21), and opposite side portions that have a temple (3) mounted pivotally and respectively thereon, said frame body (20) confining a lens opening unit which includes a pair of window openings (24), said liquid crystal cells (40) being mounted on said frame body (20) in a respective one of said window openings (24), said control circuit (5) being connected electrically to said liquid crystal cells (40).

5. The protective eyewear of Claim 4, characterized in that said lens unit further includes a pair of optical filters (41), each of which is mounted on a respective one of said liquid crystal cells (40) to provide protection against non-visible light.

6. The protective eyewear of Claim 5, characterized in that said lens unit further includes a pair of transparent protective pieces (6), each of which is mounted on one side of a respective one of said optical filters (41) opposite to a respective one of said liquid crystal cells (40).

7. The protective eyewear of Claim 1, characterized in that said control circuit (5) comprises:
a light sensing circuit (51) for generating a detected light signal that varies according to strength of ambient light; and
a liquid crystal cell driver (54) connected electrically to said light sensing circuit (51) and said liquid crystal cell (40), said liquid crystal cell driver (54) automatically generating the control signal that is applied to said liquid crystal cell (40) according to the detected light signal from said light sensing circuit (51).

8. The protective eyewear of Claim 7, characterized in that said control circuit (5) further comprises comparing means which interconnects electrically said light sensing circuit (51) and said liquid crystal cell driver (54), said comparing means receiving the detected light signal from said light sensing circuit (51) and generating an actuating signal for activating said liquid crystal cell driver (54) to generate the control signal in order to vary darkness of said liquid crystal cell (40) based on results of a comparison between the detected light signal and a pre-set reference signal.

9. The protective eyewear of Claim 7, characterized in that said control circuit (5) further comprises:
a signal shaping circuit (52) connected electrically to said light sensing circuit (51) to receive the detected light signal therefrom, said signal shaping circuit (52) processing the detected light signal to obtain a light data signal; and
a comparator (53) which interconnects electrically said signal shaping circuit (52) and said liquid crystal cell driver (54), said comparator (53) receiving the light data signal from said signal shaping circuit (52) and generating an actuating signal for activating said liquid crystal cell driver (54) to generate the control signal in order to vary darkness of said liquid crystal cell (40) based on results of a comparison between the light data signal and a pre-set reference signal.

10. The protective eyewear of Claim 9, characterized in that said light sensing circuit (51) includes a photo sensor (511) and a signal amplifier (512) connected electrically to said photo sensor (511).

11. The protective eyewear of Claim 7, characterized in that said control circuit (5) further comprises a power source (55) for providing electrical power thereto.

12. The protective eyewear of Claim 7, characterized in that said light sensing circuit (51) includes a phototransistor.

13. The protective eyewear of Claim 1, characterized in that said lens frame (3a) includes a helmet body (30) that is formed with a window opening (31), said lens unit being mounted on said helmet body (30) in said window opening (31).
